Europäisches Patentamt

European Patent Office

Office européen des brevets

(19)

(11) Veröffentlichungsnummer: **0 331 014**

**A2**

(12) # EUROPÄISCHE PATENTANMELDUNG

(21) Anmeldenummer: **89103185.8**

(22) Anmeldetag: **23.02.89**

(51) Int. Cl.⁴: **A61K 37/64**

(30) Priorität: **02.03.88 DE 3806767 U**

(43) Veröffentlichungstag der Anmeldung:
**06.09.89 Patentblatt 89/36**

(84) Benannte Vertragsstaaten:
**AT BE CH DE ES FR GB GR IT LI LU NL SE**

(71) Anmelder: **THERA - Gesellschaft für
Patentverwertung mit beschränkter Haftung
Eichenweg 4
D-8130 Starnberg(DE)**

(72) Erfinder: **Dietze, Günther, Dr.
Richildenstrasse 51
D-8000 München 19(DE)**
Erfinder: **Wicklmayr, Matthias, Dr. Dr.
Oberschlesische Strasse 15a
D-8000 München 81(DE)**

(74) Vertreter: **Freiherr von Pechmann, Eckehart et
al
Patentanwälte Wuesthoff- v.
Pechmann-Behrens-Goetz Schweigerstrasse
2
D-8000 München 90(DE)**

(54) **Verwendung von ACE-Inhibitoren für die Diabetesprophylaxe.**

(57) Die Erfindung betrifft die Verwendung der Inhibitoren des Angiotensin-Umwandlungsenzyms zur Herstellung eines Arzneimittels für die Diabetesprophylaxe bei Risikopatienten, beispielsweise bei stoffwechselgesunden Übergewichtigten. Dazu werden die Inhibitoren, beispielsweise Captopril, in einer Menge von 0,5 bis 15 mg pro Tag oral mit üblichen galenischen Hilfs- und Trägerstoffen verabriecht.

EP 0 331 014 A2

## Verwendung von ACE-Inhibitoren für die Diabetesprophylaxe

Die vorliegende Erfindung betrifft die Verwendung der Inhibitoren des Angiotensin-Umwandlungsenzyms zur Herstellung eines Arzneimittels für die Diabetesprophylaxe bei Risikopatienten.

Es ist bekannt, daß Inhibitoren des Angiotensin-Umwandlungsenzyms (Angiotensin-Converting-Enzyme oder ACE) eine starke blutdrucksenkende Wirkung beim essentiellen Hochdruck des Menschen zeigen. Derartige Inhibitoren wurden erstmals aus dem Gift der Schlange Bothrops jararaca isoliert und als Oligopeptide beschrieben; eine entsprechende Wirksamkeit wurde sowohl für das daraus erhaltene Pentapeptid mit der Bezeichnung B.P.P.$_{5a}$ als auch für das Nonapeptid der Bezeichnung B.P.P.$_{9a}$ oder SQ 20,881, letzteres mit der Aminosäurenfolge pyroGlu-Trp-Pro-Arg-Pro-Gln-Ile-Pro-Pro, nachgewiesen (siehe The Lancet 1973, 1972 und US-A-3 947 575). Ein solcher ACE-Inhibitor ist auch das Prolin-Derivat mit der chemischen Bezeichnung 1-[(2S)-3-Mercapto-2-methylpropionyl]-L-prolin, das unter der INN-Bezeichnung Captopril im Handel erhältlich ist.

Weitere ACE-Inhibitoren sind unter den Bezeichnungen Enalapril (N-(1-Ethoxycarbonyl-3-phenylpropyl)-L-alanyl-L-prolin), Lisinopril, Zofinopril und Ramipril erhältlich bzw. im arzneimittelrechtlichen Zulassungsverfahren (Zofinopril).

Die Wirkung der ACE-Inhibitoren kann mit einer Blockierung des Enzyms erklärt werden, das im Organismus das Angiotensin I in das blutdruckwirksame Angiotensin II umwandelt. Die Inhibitoren wurden bereits mit großem Erfolg bei Hochdruckpatienten angewendet, siehe New England Journal of Medicine, 1974, 817 sowie US-A-3 947 575.

Ferner ist bekannt, daß der Zusatz des ACE-Inhibitorpeptids B.P.P.$_{9a}$ bzw. SQ 20881 zu bestimmten Extraktstoffen aus dem Bluteiweiß junger Kälber zu einer Verbesserung der mit diesen Extraktstoffen erzielten Wundheilung führt. Dies bedeutet, daß die Wirkung der Extraktstoffe durch den Zusatz der ACE-Inhibitoren verstärkt wird. Die Wirkung kann darauf zurückgeführt werden, daß durch die Kombination der Extraktstoffe mit den ACE-Inhibitoren der Stoffwechsel mangelhaft versorgter Gewebspartien durch eine verstärkte Glukoseaufnahme verbessert wird, wodurch speziell bei diabetischer Stoffwechsellage die Durchblutung verstärkt und, bei lokaler Anwendung, die Wundheilung beschleunigt wird, siehe DE-A- 27 29 096 und DE- A - 27 59 793.

Die ACE-Inhibitoren wurden bisher als Mittel zur Behandlung von hypertensiven Zuständen sowie von Herzinsuffizienz verwendet sowie, in Verbindung mit Extraktstoffen aus enteiweißtem Kälberblut, zur Behandlung von Durchblutungs- und Stoffwechselstörungen.

Es wurde nun überraschend gefunden, daß ACE-Inhibitoren bei oraler Verabreichung in Mengen, die den Blutdruck nicht mehr wesentlich beeinflussen, an stoffwechselgesunden, nicht diabetische Risikopatienten die Erkrankungsrate an Diabetes mellitus signifikant zu vermindern in der Lage sind.

Unter stoffwechselgesunden, nicht diabetischen Risikopatienten werden insbesondere solche Patientengruppen verstanden, die wegen eines erhöhten Körpergewichts und/oder erblicher Veranlagung und/oder anderer Einflußfaktoren eine erhöhte Erkrankungsrate an Diabetes mellitus erwarten lassen. So liegt beispielsweise bei stark übergewichtigen Patienten mit einem Alter von mehr als 50 Jahren das Risiko, an Diabetes mellitus zu erkranken, bei etwa 45 %, bezogen auf einen Zeitraum von zwei Jahren. Bei solchen Risikogruppen führte die Einnahme von, beispielsweise, 2 x 3 mg Captopril pro Tag zu einer Verminderung dieses Risikos um mehr als 50 %.

Bei der Behandlung mit den erfindungsgemäß verwandten ACE-Inhibitoren handelt es sich um eine reine Prophylaxe; die tägliche Dosierung an ACE-Inhibitor reicht zu einer wesentlichen Beeinflussung des Blutdrucks nicht aus. Die Verabreichung von ACE-Inhibitoren führt auch nicht zu einer Senkung des Blutzuckerspiegels und hat keinen Einfluß auf die Insulinausschüttung.

Diese Wirkung der ACE-Inhibitoren an Gesunden war nicht vorhersehbar, da den Glukosestoffwechsel verbessernde Medikamente wie Sulfonylharnstoffe, Biguanide oder Insulin für eine solche prophylaktische Behandlung nicht geeignet sind. Die Verabreichung von Sulfonylharnstoffen hat beispielsweise einen Einfluß auf die Insulinausschüttung und würde zudem zu Übergewicht führen, d.h. die Gewichtsprobleme der Patienten dieser Risikogruppe weiter verschärfen.

Für die erfindungsgemäße Verwendung werden die ACE-Inhibitoren in Mengen von 0,5 bis 15 mg/d, vorzugsweise 1 bis 10 mg/d oral verabreicht. Eine typische Dosierung für einen übergewichtigen Erwachsenen ist beispielsweise 2 x 3 mg Captopril pro Tag, jedoch kann die Dosierung in Abhängigkeit vom Körpergewicht, Alter und physischen Zustand des Patienten in üblicher Weise variiert werden.

Der Wirkstoff für die erfindungsgemäße Verwendung wird in für die orale Anwendung üblicherweise mit gängigen Hilfs- und Trägerstoffen formuliert. Dabei liegt die Einzeldosis vorzugsweise im Bereich von 0,5 bis 10 mg, insbesondere 1 bis 5 mg, ACE-Inhibitor.

Bei der Behandlung von Bluthochdruck beträgt die Tagesdosis an Captopril dagegen beispielsweise bei 25 mg bis 150 mg pro Tag; sie liegt also weit über der für die Diabetesprophylaxe benötigten täglichen Menge.

Die Diabetes -prophylaktische Wirkung der ACE-Inhibitoren ist aus dem nachfolgend beschriebenen Versuch, der mit Captopril durchgeführt wurde, und dessen Ergebnissen ersichtlich.

Versuchsbericht

Es wurden 122 übergewichtigte Personen ausgesucht, die im Mittel 25 % Übergewicht und eine gestörte orale Glukosetoleranz hatten. Diese Patienten gaben nach Aufklärung ihr Einverständnis an dieser Studie über 3 Jahre teilzunehmen. Sie hatten bereits 5 bis 10 Versuche einer Gewichtsabnahme hinter sich, die alle erfolglos verlaufen waren. Ein Diabetes mellitus war nach der Definition des World Health Organization-Reports (Techn. Rep. Ser. 646, 1-80, 1980), bei diesen Patieten ausgeschlossen. Sie wurden in zwei gleich starke Gruppen eingeteilt, so daß nach Alter, Ausmaß der Störung des Glucose-Toleranz-Tests (OGTT) und Größe und Dauer des Übergewichts kein Unterschied bestand. Die Patienten wurden in halbjährlichen Abständen ambulant kontrolliert. Wie zu Beginn, wurden bei diesen Kontrollen folgende Blutwerte gemessen: Blutsenkung, Blutbild, Nüchternblutzucker, OGTT, Gewicht, Cholesterin und Triglyzeride, Serum Glutamat-Oxalat-Transaminase (SGOT), Serum Glutamat-Pyruvat Transaminase (SGPT). Damit wurden den Stoffwechesl beeinträchtigende Krankheiten ausgeschlossen.

Sie erste Gruppe erhielt 2 x 3 mg Captopril täglich, die zweite entsprechend Placebo. Die Patienten hatten die Auflage, keine den Stoffwech l beeinträchtigende Medikamente, z.B. Diuretika, $\beta$-Blocker ode dergleichen einzunehmen. In beiden Gruppen schieden über den Zeitraum von 3 Jahren 28 Patienten aus verschiede nen Gründen aus. Die Gruppen veränderten sich dadurch jedoch nicht so, daß sie statistisch nicht mehr auswertbar waren.

Unter Anwendung der Student's-t-Tests (Snedecor, G.W.; Cochran, W.E.: Statistical methods, II[th] Edition, Ames Iowa: Iowa-State, Universitiy-Press, 1967) der Mantel-Haenszel-Gleichung (Mantel, N.: J.Am. Stat. Ass. 58: 690-700 (1963), Miettinen, O.: Am. J. Epidemiol. 103: 226-235 (1976)) und Pitman's Permutationstests (Bradley, J.V. Distribution-free statistical tests. Englewood Cliffs, New Jersey: Prentice-hall 1968, 68-86) ergab sich unter Berücksichtigung der vorliegenden variablen Hypertriglyzeridämie und familiäre Diabetesbelastung nach 3 Jahren eine signifikant geringere Erkrankungsrate an Diabetes mellitus in der Captopril-Gruppe (p < 0,001: Student's t-Test).

Die Ergebnisse des Versuchs sind in der nachstehenden Tabelle dargestellt.

ERKRANKUNGSRATE AN DIABETES MELLITUS BEI ÜBERGEWICHTIGEN FRAUEN[a]

UNTER CAPTOPRIL (C)[b] GEGENÜBER PLACEBO (P).

| ZEITRAUM | | 1985 -1 | 1986 - 2 | 1987 - 2 | 1988 - 1 |
|---|---|---|---|---|---|
| ANZAHL | C | 63 | 60 | 54 | 48 |
| | P | 59 | 54 | 49 | 46 |
| ALTER | C | 55,3 ± 0,8 | 56,2 ± 0,9 | 57,4 ± 0,9 | 58,6 ± 1,0 |
| (Jahre) | P | 57,2 ± 1,1 | 58,4 ± 1,0 | 59,6 ± 1,3 | 60,3 ± 1,3 |
| Gewicht | C | 126,0 ± 1,1 | 124,1 ± 1,6 | 125,2 ± 1,1 | 125,5 ± 1,6 |
| (in % des Idealgewichts[c]) | P | 124,1 ± 1,3 | 136.0 ± 1,4 | 123,6 ± 2,1 | 124,1 ± 1,9 |
| DIABETES | C | 0 | 2 | 4 | 4 |
| MELLITUS[d] | P | 0 | 3 | 6 | 11[e] |

a mit gestörtem OGTT; keine diabetogenen Medikamente

b 2 X 3 mg tgl.

c Idealgewicht: Größe (cm)-100-15%

d WHO-Report: Techn. Rep. Ser. 646, 1-80 (1980)

e Signifikanz bei p< 0,001;Student's t-Test. Sie
wurde nach Mantel-Haenszel[1]u. Miettinen[2] kalkuliert
u. bleibt erhalten wenn Hypertriglyzeridämie u.
familiäre Diabetesbelastung berücksichtigt werden[3].

1 Mantel, N.J.Am.Stat.Ass. 58: 690-00 (1963)

2 Miettinen,O., Am.J.Epidemiol. 103: 226-235
(1963)

3 Bradley,J.V., Distribution-free statistical tests. Englewood Cliffs, New Jersey:
Prentice-Hall 1968, 68-86.

EP 0 331 014 A2

## Ansprüche

1. Verwendung der Inhibitoren des Angiotensin-Umwandlungsenzyms zur Herstellung eines Arzneimittels für die Diabetesprophylaxe bei Risikopatienten.

2. Verwendung nach Anspruch 1, gekennzeichnet durch eine Menge von 0,5 bis 10 mg Wirkstoffe je Dosierungseinheit.